# EUROPÄISCHE PATENTSCHRIFT

(11) **EP 1 781 807 B1**
(45) Veröffentlichungstag und Bekanntmachung des Hinweises auf die Patenterteilung: **03.12.2008**
(21) Anmeldenummer: 05768798.0
(22) Anmeldetag: 05.08.2005
(51) Int. Cl.: C12Q 1/52, C12Q 1/56

(54) **FLUORESZENZBASIERENDE KINETISCHE BESTIMMUNG DER AKTIVITÄT VON TRANSGLUTAMINASEN**
FLUORESCENCE-BASED KINETIC DETERMINATION OF TRANSGLUTAMINASE ACTIVITY
DETERMINATION CINETIQUE FONDEE SUR LA FLUORESCENCE DE L'ACTIVITE DE TRANSGLUTAMINASES

(30) Priorität: 11.08.2004 EP 04019090; 20.08.2004 US 603374 P
(43) Veröffentlichungstag der Anmeldung: 09.05.2007
(73) Patentinhaber: N-Zyme BioTec GmbH, 64295 Darmstadt (DE)
(72) Erfinder: PASTERNACK, Ralf, 64347 Griesheim (DE); OERTEL, Kai, 55122 Mainz (DE)
(74) Vertreter: Arth, Hans-Lothar
(86) Internationale Anmeldenummer: PCT/EP2005/008532
(87) Internationale Veröffentlichungsnummer: WO 2006/018164

(56) Entgegenhaltungen:
- PARAMESWARAN KUMARAPURAM N ET AL: "Hydrolysis of gamma:epsilon isopeptides by cytosolic transglutaminases and by coagulation factor XIIIa" JOURNAL OF BIOLOGICAL CHEMISTRY, Bd. 272, Nr. 15, 1997, Seiten 10311-10317, XP002301670 ISSN: 0021-9258 in der Anmeldung erwähnt
- PASTERNACK R ET AL: "A fluorescent substrate of transglutaminase for detection and characterisation of glutamine acceptor compounds" ANALYTICAL BIOCHEMISTRY, ACADEMIC PRESS, SAN DIEGO, CA, US, Bd. 249, 1997, Seiten 54-60, XP002090480 ISSN: 0003-2697

## Beschreibung

Die vorliegende Erfindung betrifft ein fluoreszenzbasierendes Verfahren zur qualitativen und quantitativen Bestimmung der Aktivität von Transglutaminasen unter Verwendung modifizierter Peptidsubstrate, neue fluoreszenzmarkierte Peptidsubstrate sowie die Verwendung solcher Verfahren und/oder Substrate in Screeningassays.

Im menschlichen Körper wurden bis heute neun verschiedene Transglutaminasen identifiziert. Transglutaminasen spielen eine immer größer werdende Rolle in der medizinischen Diagnostik, nachdem eine wachsende Anzahl von Krankheiten mit Transglutaminasen in Verbindung gebracht wurden (Gentile, V., et al., Current Drug Targets - CNS & Neurological Disorders, 3, 2004, 69-74; Kim, S.Y., et al., Neurochem Int. 2002, 85-103, Cooper A. J., Neurochem Int., 2002, 1-5). Bereits heute wird in der klinischen Routinediagnostik die Messung des Blutgerinnungsfaktors XIII (FXIII) durchgeführt, da mit einem Mangel dieser Transglutaminase ernsthafte Erkrankungen verbunden sind (Klinische Aspekte des Faktor-XIII-Mangels, Karger Verlag, 1999, Hrsg.: R. Egbring, R.Seitz, G. Wozniak).

Obwohl eine Vielzahl von Aktivitätstests für Transglutaminasen bekannt sind, existiert bis heute kein Verfahren, welches allgemein anwendbar, einfach und schnell in der Durchführung ist, eine hohe Sensitivität besitzt und gleichzeitig eine hohe Selektivität zwischen den verschiedenen Transglutaminasen gewährleistet.

Im Wesentlichen werden zur quantitativen Messung die folgenden Methoden benutzt: der Einbau eines markierten Amins in ein Protein und die direkte oder indirekte Quantifizierung des Markers, z. B. der Einbau von Dansylcadaverin in N,N-Dimethylcasein (US 4,601,977, Lorand L. et al., J. Clin. Invest.,48, 1969, 1054-64) bzw. von Biotinylcadaverin in N,N-Dimethylcasein (US 5,015,588; Lee K.N. et al., Clin. Chem., 34, 1988, 906-10; Song Y.C. et al., Anal. Biochem., 223, 1994, 88-92), sowie Radioisotopen (Dviansky A. et al., Br. J. Haematol. 18, 1970, 399-410; Lorand L. et al., Anal. Biochem., 50, 1972, 623-31). Eine andere quantitative Methode misst die Menge des gebildeten Ammoniaks, die durch Hydrolyse bzw. Aminolyse eines zugesetzten synthetischen Peptids entsteht (US 5,204,240, Clin. Chem., Vol. 31, Nr. 1, 1985, 35-40).

Andere Methoden sind speziell auf eine bestimmte Transglutaminase gerichtet, wie zum Beispiel die Messung des Vernetzungsgrades von Fibrin zur Bestimmung der Aktivität des Faktors FXIII (US 5,508,202 und US 6,406,874; siehe auch Karges, R., Clemens R., Behring Inst. Mitt. 82, 1988, 43-58). Neben diesen Testverfahren sind in der wissenschaftlichen Literatur eine Vielzahl von Verfahren zur Messung der Transglutaminase-Aktivität bekannt. (Grossowicz, J. Biol. Chem. 187, 1950; Lorand L., Anal. Biochem., 1971, 44, 221-231; Slaughter et al., Anal Biochem. 1992, 205(1):166-71; Aeschliman et al., J. Biol. Chem. 1995; 270(40):23415-20; 111-125; Lorand L., J. Biol. Chem. 1997, 10311-10317; Pasternack R. et al., Anal Biochem. 1997;249(1):54-60; Lorand L., Exp- Eye Res. 1998, 66, 531-536; Keillor J.W. et al., Anal Biochem. 1999 274(1):141-4; Keillor J.W. et al., Anal Biochem. 2000, 285(1):16-20; Furutani et al., J Histochem Cytochem. 2001 Feb;49(2):247-58; Jeitner T.M., Anal Biochem. 2001;292(2):198-206; Fesus L. et al., Anal Biochem. 2002, 311 (2):187-90; Keillor J.W., Biochemistry. 2003;42(39):11504-13)

Gemeinsam ist diesen Verfahren, dass sie zur Beantwortung bestimmter wissenschaftlicher Fragestellungen in der Grundlagenforschung geeignet sind. In der Handhabung und Durchführung sind sie jedoch zu zeitaufwändig und können zudem nicht zwischen verschiedenen Transglutaminasen unterscheiden. Auch die Genauigkeit der Messungen ist insbesondere bei geringen Transglutaminaseaktivitäten häufig nicht ausreichend. Gerade geringe Aktivitäten von Transglutaminasen sind aber in der medizinischen Diagnostik von entscheidender Bedeutung, da solche Mangelzustände (< 5 % des normalen physiologischen Levels), genetische Defekte und Polymorphismen erhebliche Relevanz für die betroffenen Patienten besitzen.

Parameswaran et al. beschreiben die kinetische Messung der Transglutaminaseaktivität mittels der Hydrolyse von fluoreszenzmarkierten Peptiden, in denen das eigentliche Substratglutamin durch eine in der 5-Position mit einer die Fluoreszenz quenchenden Molekülgruppe (2,4-Dinitrophenylcadaverin) substituierten Glutaminsäure ersetzt wird (Parameswaran et. al., J. Biol. Chem., 1997, 10311-17). Als Fluorophor finden N-[5-(Dimethylamino)-1-naphtalin-sulfonsäure]-ε-aminohexansäure bzw. N-(2-Aminobenzoyl)-ε-aminohexansäure Verwendung. Gemessen wird dabei die zunehmende Fluoreszenz im Ansatz, die durch die Hydrolyse der Seitenkette und damit der räumlichen Entfernung des Fluoreszenzquenchers aus dem fluoreszierenden Peptid bedingt ist. (Zur Theorie des zugrunde liegenden Förster-Energie-Transfers siehe z. B. Szöllosi J., Cytometry, 34, 1998, 159-179). Die steigende Fluoreszenz kann als Maß für die Aktivität der Transglutaminase nachgewiesen werden. Parameswaran et al. setzen ausschließlich solche Moleküle ein, die das Fluorophor über einen Alkanoylspacer (hier 6-Aminohexansäure) am N-terminalen Ende der Peptidsequenz tragen. Als Basis der Peptidsequenz dient hierbei ausschließlich Gln-Gln-Ile-Val, wobei das N-terminale Glutamin in oben genannter Weise modifiziert wird.

Nachteilig an dem oben genannten System ist die Tatsache, dass die Menge der Transglutaminase, die eingesetzt werden muss, um einen nachweisbaren Anstieg der Fluoreszenz zu erreichen z. B. für FXIIIₐ mit 100 mg/Liter wesentlich über den Konzentrationen liegt, die physiologisch relevant sind (ca. 10 bis 15 mg/Liter). Die Autoren erzielen eine nur 1,5fache Steigerung des Ausgangswertes (Fig. 7) und benötigen eine Konzentration des Faktors XIIIₐ (von den Autoren rA₂ genannt) von 8 µM, was 1,36 mg des Faktors XIIIₐ pro ml Blut entspricht. In menschlichem Blut liegt der nachzuweisende Faktor jedoch nur in 10-15 µg pro ml Blut vor und die unvermeidbare Verdünnung des Patientenplasmas mit Substratlösung verringert die für den Nachweis zur Verfügung stehende Konzentration dieses Faktors auf 1 bis 1,5 µg Faktors XIIIₐ pro ml Assayvolumen. Ein für die in Blut tatsächlich vorliegenden physiologischen Bedingungen geeigneter Assay bedarf daher einer Empfindlichkeit, die 1000 x größer als die mit dem Verfahren von Parameswaran et al. erreichte.

In Anbetracht der geschilderten Nachteile des Standes der Technik besteht die Aufgabe der vorliegenden Erfindung in der Bereitstellung eines neuen selektiven Verfahrens zur schnellen, einfachen und vor allem hochempfindlichen kinetischen Messung von Transglutaminase-Aktivitäten, das auch für klinische Anwendungen geeignet ist.

Diese Aufgabe wird erfindungsgemäß durch ein auf Fluoreszenz basierendes Verfahren zur Bestimmung der Aktivität von Transglutaminasen unter Verwendung modifizierter Peptidsubstrate gemäß Anspruch 1 gelöst.

Dabei werden modifizierte Peptide eingesetzt, die neben einem Fluorophor, das direkt an die N-terminale Aminogruppe des Peptids gebunden ist, eine Molekülgruppe in der 5-Position eines Glutaminsäurerestes tragen, welche die Fluoreszenz des Fluorophors weitgehend unterdrückt (Fluoreszenzquencher). Gemessen wird der Anstieg der Fluoreszenz durch die hydrolytische oder aminolytische Abspaltung des Fluoreszenzquenchers durch die Aktivität der Transglutaminase.

Bei der Entwicklung des erfindungsgemäßen Verfahrens wurde überraschenderweise festgestellt, dass die molekulare Struktur sowie die Art der Anbindung des Fluorophors einen entscheidenden Einfluss auf die Substrateigenschaften des Peptidsubstrats und die Signalintensität bei der Aktivitätsmessung haben. Durch die direkte Bindung von z. B. 2-Aminobenzoesäure (Abz) oder strukturell ähnlichen Verbindungen an den N-Terminus einer geeigneten Peptidsequenz werden Peptide erhalten, die abhängig von der verwendeten Peptidsequenz, von verschiedenen Transglutaminasen außergewöhnlich gut als Substrat erkannt werden. Die notwendige Konzentration des Substratpeptids ist überraschend niedrig und liegt in der Regel im Bereich von 10-100 µM. Die guten Substrateigenschaften dieser fluoreszenzmodifizierten Peptide ermöglichen auch die Messung sehr geringer Transglutaminase-Aktivitäten, wie sie unter physiologischen Bedingungen häufig vorkommen, und die bisher so nicht messbar waren.

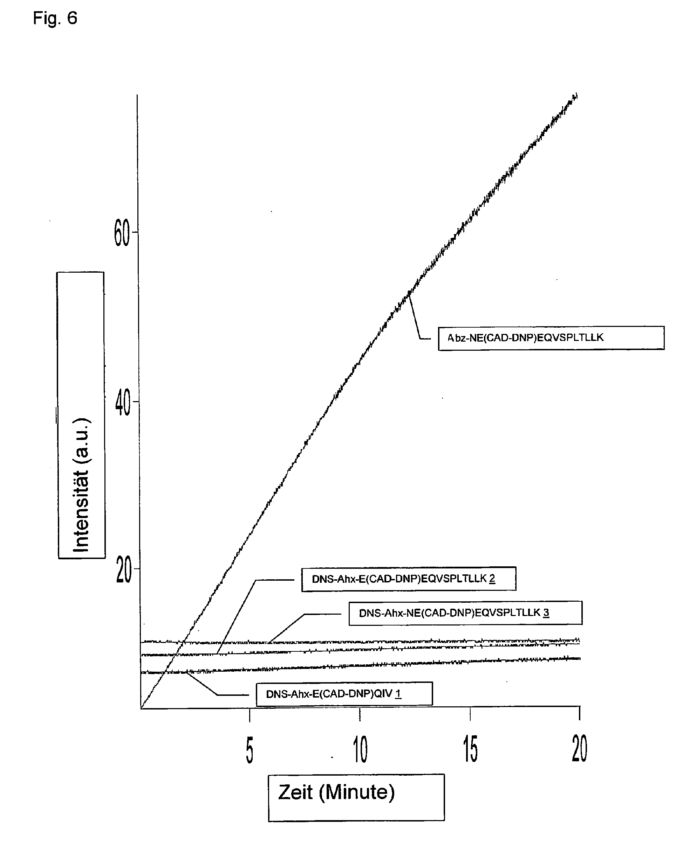

Die überraschenden und vorteilhaften Eigenschaften der in dem erfindungsgemäßen Verfahren eingesetzten Substrate zeigen sich besonders deutlich im direkten Vergleich der Verbindungen des Standes der Technik gegenüber routinemäßig veränderten Derivaten und erfindungsgemäß veränderten Derivaten bei ansonsten gleicher Primärstruktur und unter ansonsten gleichen Assaybedingungen. Wie in Beispiel 5 und der dazu korrespondierenden grafischen Auswertung in Fig. 6 weiter hinten gezeigt wird, führt im Gegensatz zu den von Parameswaran et al. beschriebenen fluoreszenzmarkierten Substraten der Transglutaminase mit 6-Aminohexansäure als Abstandsgruppe zum Fluoreszenzmarker (Verbindung 1) unter physiologisch relevanten Faktor XIIIa Konzentrationen nur die direkte Bindung der N-terminalen Aminosäure an das Fluorophor zu einem deutlichen und leicht messbaren Anstieg der Fluoreszenz. Dahingegen führen andere routinemäßige Verbesserungsmaßnahmen an den im Stand der Technik bekannten fluoreszenzmarkierten Peptidsubstrate wie die Optimierung der von der Transglutaminaase zu spaltenden Peptidsequenz oder das Ersetzen des Fluorophors zu keiner sinnvollen Verbesserung der Nachweisempfindlichkeit.

Die durch die Verwendung eines direkt an das Peptidsubstrat gebundenen Fluorphors bedingte überraschende Steigerung der Nachweisempfindlichkeit von Transglutaminase-aktivitäten ist schwer zu erklären. Dem Fachmann ist zwar bekannt, dass verschiedene Faktoren für eine kinetische Messung einer Enzymaktivität durch modifizierte Peptide basierend auf der FRET-Technologie maßgeblich sind. Als kritischer Faktor ist hierbei zum einen der Abstand der beiden Farbstoffe anzusehen, da der FRET-Effekt stark abstandsabhängig ist. Zum anderen muss die Peptidstruktur eine ausreichende Enzymumsetzung gewährleisten. Im Rahmen der Untersuchungen der Erfinder (siehe Beispiel 5) hat sich aber gezeigt, dass die beiden zuvor genannte Faktoren für die hohe Empfindlichkeit des erfindungsgemäßen Nachweisverfahrens eindeutig nicht maßgeblich verantwortlich sind. Beim Vergleich des maximalen Abstands der Farbstoffe in den im vergleichenden Beispiel 5 eingesetzten Peptidsubstraten stellt man fest, dass sich der Abstand der Farbstoffe im Stand der Technik Abz-Ahx-Glu(CAD-DNP) zu dem erfindungsgemäßen Peptidsubstrat Abz-Asn-Glu(CAD-DNP) letztendlich nur um eine Bindungslänge unterscheidet (20 vs 19 Bindungen). Wie im Vergleich nachgewiesen wurde, ist also weder der Abstand der Farbstoffe in dem erfindungsgemäßen Peptidsubstrat noch das jeweilige Peptidrückgrat für die außergewöhnliche Empfindlichkeit der erfindungsgemäßen Substrate verantwortlich. Letztendlich ist die erfindungsgemäße Wirkung ausschließlich der Tatsache der direkten Bindung des Fluorophores an den N-Terminus zuzuschreiben. Dieses verbesserte Wirkungsprinzip war nicht nur bisher vollkommen unbekannt, es kann auch ohne Einschränkungen auf beliebige durch Fluoreszenzmarker markierte Transglutaminasepeptidsubstrate übertragen werden.

Ein weiterer Vorteil des erfindungsgemäßen Verfahrens gegenüber dem Stand der Technik sind neben der Empfindlichkeit der Messung die Tatsache, dass damit eine diskriminierende Messung verschiedener Transglutaminasen möglich ist. Das bedeutet, dass ein Substrat, welches hinsichtlich der verwendeten Peptidsequenz für eine bestimmte Transglutaminase geeignet ist, von einer anderen Transglutaminase nicht oder nur sehr geringfügig umgesetzt wird. Sind beide Transglutaminasen zum Beispiel in einem Zellhomogenisat vergesellschaftet, so kann ihre Aktivität unabhängig voneinander gemessen werden.

Als Fluorophor kommen grundsätzlich alle Fluorophore in Frage, welche die gewünschte hohe Sensitivität des erfindungsgemäßen Verfahrens gewährleisten. Vorzugsweise handelt es sich dabei um am Benzolring substituierte Benzoesäurederivate, bevorzugter um Benzoesäurederivate, die an der 2-Position einen Substituenten tragen, besonders bevorzugt sind 2-Aminobenzoesäure und N-Methyl-2-aminobenzoesäure. "Direkte Bindung des Fluorophors" bedeutet im Kontext der vorliegenden Erfindung, dass sich keine Spacergruppierung zwischen dem Fluorophor und dem N-Terminus des Peptids befindet. So besteht z. B. im Falle der 2-Aminobenzoesäure eine direkte Amidbindung zwischen der Carboxylgruppe der Benzoesäure und der Aminofunktion des Peptids.

Der Fluoreszenzquencher muss hinsichtlich der spektralen Eigenschaften auf das Fluorophor abgestimmt sein und zueinander passende Paare von Fluorophor und Fluoreszenzquencher sind seit langem bekannt und werden häufig verwendet. Geeignete Partner für die erfindungsgemäß besonders bevorzugte 2-Aminobenzoylgruppierung sind Fluoreszenzquencher, die z. B. eine 2,4-Dinitrophenol- oder 3-Nitrotyrosingruppe enthalten bzw. daraus bestehen (vgl. z. B. Juliano, M.A., et al., J. Pept.Res., 53, 1999, 109-19).

Als Peptidsubstrat werden niedermolekulare Peptide eingesetzt, in denen das Glutamin, welches als Substrat-Aminosäure für die jeweilige Transglutaminase dient, durch eine Glutaminsäure ersetzt wird, die einen Fluoreszenzquencher trägt, welcher über einen Spacer, der eine Aminfunktion enthält, unter Ausbildung einer Amidbindung an die 5-Position der Glutaminsäure gebunden ist. Vorzugsweise ist dieser Spacer ein Alkylaminspacer, der in der Regel 1 bis 6, vorzugsweise 5, Methylengruppen zwischen Fluoreszenzquencher und Glutaminsäure enthält. Die zweite funktionelle Gruppe des Spacers zur Anbindung des Fluoreszenzquenchers kann in Abhängigkeit von der Molekülstruktur des speziell eingesetzten Fluoreszenzquenchers variierten, Insbesondere für Fluoreszenzquencher, die eine 2,4-Dinitrophenol- oder 3-Nitrotyrosingruppe enthalten, werden vorzugsweise 1,n-Alkydiaminspacer, besonders bevorzugt 1,5-Pentandiamin (Cadaverin, abgekürzt CAD) verwendet. Vorzugsweise werden als Substratpeptide solche Peptide eingesetzt, die selektiv für die zu messende Transglutaminase sind.

In einer bevorzugten Ausführungsform wird das Peptidsubstrat durch die Formel I repräsentiert 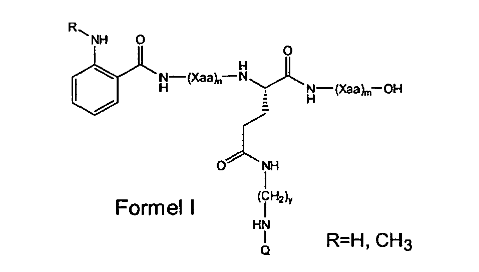
worin Xaa ein beliebige Aminosäure oder ein Aminosäurederivat davon ist,
n und und m unabhängig voneinander 0 bis 10 sind,
y 1 bis 6, vorzugsweise 5, ist,
Q ein Fluoreszenzquencher ist,
R H oder CH₃ ist.

Die neben dem modifizierten Glutaminsäurerest vorhandenen Aminosäuren des Peptidsubstrates, Xaa in der Formel I, können eine beliebige Aminosäure, vorzugsweise eine natürliche Aminosäure, oder ein die Substrateigenschaften nicht wesentlich beeinträchtigendes Derivat davon sein.

Geeignete, jedoch nicht beschränkende Beispiele für erfindungsgemäße selektive Peptidsubstrate sind Abz-Asn-Glu((CH₂)_{y}-NH-Q)-Glu-Gln-Val-Ser-Pro-Leu-Thr-Leu-Leu-Lys und Abz-Pyr-Ala-Glu((CH₂)_{y}-NH-Q)-Gln-Ile-Val für FXIII sowie Abz-Ala-Pro-Glu((CH₂)_{y}-NH-Q)-Gln-Glu-Ala für humane Gewebetransglutaminase (Transglutaminase 2) und die entsprechenden N-Methyl-Abz-Derivate davon.

Das erfindungsgemäße Verfahren kann zur Aktivitätsmessung jeder bekannten Transglutaminase genutzt werden, denn alle Mitglieder der Enzymfamilie katalysieren grundsätzlich dieselbe Reaktion. Bevorzugt sind bakterielle Transglutaminasen und Transglutaminasen aus Vertebraten, bevorzugter Säuger-Transglutaminasen, besonders bevorzugt humane Transglutaminasen. Nicht beschränkende Beispiele geeigneter humaner Transglutaminasen sind Faktor XIII, humane Transglutaminase 2, TG3, TG4, TG5, TG6, TG7.

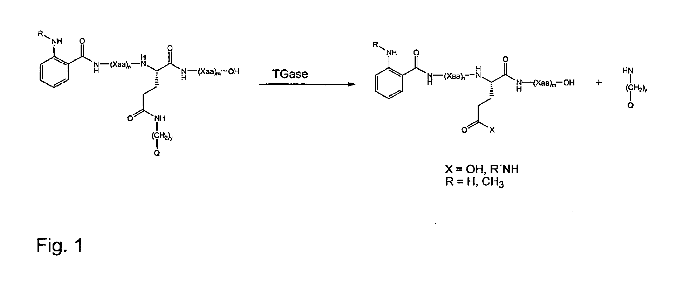

Eine typische Ausführungsform des erfindungemäßen Verfahrens ist die selektive Bestimmung von verschiedenen Transglutaminasen in Transglutaminase enthaltenden Proben, beispielsweise FXIII in Plasmen oder TG2 in Gewebeproben. Je nach Art der biologischen Probe können dabei zusätzliche Ingredienzien und Hilfsstoffe oder Vorbereitungsschritte von Vorteil sein. Zum Beispiel wird in unbehandelten Plasmen FXIII günstiger Weise mit Thrombin aktiviert und ein Fibrinaggregationshemmer, vorzugsweise Gly-Pro-Arg-Pro-amid, zugesetzt. Bei anderen Proben könnte der Zusatz anderer Additive vorteilhaft oder notwendig sein. Dadurch wird jedoch die breite Einsetzbarkeit der erfindungsgemäßen modifizierten Peptidsubstrate, insbesondere der allgemeinen Formel I, nicht eingeschränkt. In einer bevorzugten Ausführungsform wird dem Reaktionsansatz ein primäres Amin zugegeben, wodurch die Hydrolysereaktion zu einer Aminolysereaktion wird (X=RHN in Fig. 1). Dadurch kann die Signalstärke zusätzlich erhöht werden.

Einige geeignete, nicht beschränkende, Beispiele für solche Amine sind ein Glycinmethyl- oder -ethylester, Ethanolamin, Diaminoethan oder ein Derivat von 1,5-Diaminopentan.

Mit dem erfindungsgemäßen Verfahren kann eine Bestimmung der Transglutaminase-Aktivität in Proben schnell und sicher durchgeführt werden. Die Testausführung beinhaltet vorzugsweise folgende Schritte:
- Vorlegen der zu untersuchenden Probe;
- gegebenenfalls Zusatz von Aggregationshemmern oder anderen Hilfsstoffen, die gegebenenfalls in einer Pufferlösung vorliegen;
- gegebenenfalls Aktivierung einer Transglutaminase, z. B. FXIII durch Thrombin, und Aktivierung für einen gegebenen Zeitraum. Die Aktivierung kann z. B. in einer bereits Calciumionen-haltigen Lösung durchgeführt werden oder den Zusatz von Calciumionen beinhalten.
- Zusatz von Substratlösung, die entweder in Pufferlösung oder in einem Gemisch aus Dimethylsulfoxid oder einen anderen geeignetem Lösungsmittel und Pufferlösung gelöst vorliegt. Vorzugsweise liegt das Substrat in einer Konzentration von 10 bis 100 µM im Endansatz vor.
- Messen der Fluoreszenzänderung.

Vorzugsweise sollten alle Lösungen vor Beginn der Messung auf eine Temperatur gebracht werden, bei der mit einer hohen Aktivität der Transglutaminase gerechnet werden kann. Eine für die meisten bekannten Transglutaminasen geeignete Temperatur beträgt etwa 37°C. Erforderlichenfalls kann die optimale Assay-Temperatur für eine bestimmte Transglutaminase von einem Fachmann durch Routineversuche festgestellt werden.

Die erfindungsgemäße Aktivitätsmessung kann sowohl als Einzelmessung erfolgen wie auch als parallele Messung mehrerer Proben in einer dazu geeigneten Vorrichtung für parallelisierte Messverfahren. Solche Vorrichtungen sind im Stand der Technik an sich bekannt und können leicht an das erfindungsgemäße Verfahren adaptiert werden. In einer speziellen Ausführungsform umfasst die Vorrichtung Mikrotiterplatten, in denen die Messungen vorgenommen werden können, und entsprechende Instrumente zur Messung der Fluoreszenz.

Neben diagnostischen bzw. klinischen Anwendungen und in der Grundlagenforschung eignen sie die erfindungsgemäßen Verfahren auch besonders gut für Screeningassays zur Identifizierung von Transglutasminasemodulatoren, insbesondere für High Throughput Screening (HTS) Assays, wie sie in der pharmazeutischen Industrie routinemäßig zur Wirkstofffindung in Bezug auf spezielle "Targets" eingesetzt werden. Bei diesen HTS-Assays werden beispielsweise 96er/384er/etc. Wellplatten eingesetzt. Solche erfindungsgemäßen Verfahren bestimmen den Einfluss von Verbindungen auf die Aktivität der eingesetzten Transglutaminase und charakterisieren die untersuchten Verbindung je nach Ergebnis als kein Modulator (keine Änderung der Aktivität im Vergleich zu einer Versuchskontrolle), Aktivator der Aktivität (stärkere Aktivität im Vergleich zu einer Versuchskontrolle) oder aber Hemmer der Aktivität (geringere Aktivität im Vergleich zu einer Versuchskontrolle) der eingesetzten Transglutaminase. Als Versuchskontrolle bzw. zur Referenzwertbestimmung kann ein bekannter Hemmer, Aktivator oder die Abwesenheit einer die Transglutaminaseaktivität modulierenden Verbindung eingesetzt werden. Der Versuchsaufbau von Verfahren der vorliegenden Erfindung als Screeningassay hängt im Detail von der jeweiligen eingesetzten Transglutaminase ab und der Fachmann kann die Bedingungen solcher erfindungsgemäßer Assays ohne Weiteres routinemäßig mit geringem Aufwand bestimmen bzw. optimieren.

Daher betrifft in einer bevorzugten Ausführungsform die vorliegende Erfindung auch ein fluoreszenzbasierendes Verfahren zur Identifizierung von Verbindungen, die eine Transglutaminase-Aktivität modulieren, umfassend das Inkontaktbringen von wenigstens eine Verbindung mit einer Transglutaminase und die anschließende Messung der Transglutaminase-Aktivität gemäß einem der Verfahren der Anspruche 1 bis 16.

Vorzugsweise betrifft die Erfindung insbesondere ein Verfahren, bei dem Verbindungen identifiziert werden, die eine Transglutaminase-Aktivität verstärken oder verringern, mehr bevorzugt die Transglutaminase-Aktivität verringern.

Ein weiterer Aspekt der vorliegenden Erfindung ist auf die Verwendung der erfindungsgemäßen Peptidsubstrate in einem Verfahren zur fluoreszenbasierenden kinetische Messung von Transglutaminase-Aktivität gerichtet.

Vorzugsweise werden erfindungsgemäße Peptidsubstrate in einem erfindungsgemäßen Verfahren verwendet.

Zudem umfasst die Erfindung die Verwendung eines erfindungsgemäßen Verfahrens zur Identifizierung von Modulatoren der Aktivität einer Transglutaminase.

### FIGURENBESCHREIBUNG:

**Fig. 1** Durch Transglutaminase katalysierte Abspaltung eines Fluoreszenzquenchers von einem fluoreszenzmodifizierten Peptidsubstrat
**Fig. 2** Anregungs- und Emissisionspektrum der nach dem Synthesebeispiel 1 erhaltenen Substratverbindung 2
**Fig. 3** Fluoreszenzänderung der Substratverbindung 2 mit der Zeit nach Umsetzung mit FXIII unter den in Beispiel 1 angegebenen Reaktionsbedingungen
**Fig. 4** Fluoreszenzänderung der Substratverbindung 3 mit der Zeit nach Umsetzung mit humaner Transglutaminase 2 unter den in Beispiel 2 angegebenen Reaktionsbedingungen
**Fig. 5** Fluoreszenzänderung der Substratverbindung 3 mit der Zeit nach Umsetzung mit Meerschweinchenleber-Transglutaminase unter den in Beispiel 3 angegebenen Reaktionsbedingungen
**FIG. 6** Vergleich der Fluoreszenzänderung von (i) Substratverbindungen des Standes der Technik mit 6-Aminohexansäure (Ahx) als Spacer zwischen dem N-Terminus des Peptids und den Fluorophoren Dansyl (DNS) und 2-Aminobenzoesäure (Abz) (Parameswaran et al., Verbindung 1), solchen mit verbesserter Transglutaminasespezifität (Verbindung 2), solchen mit am N-Terminus veränderter Aminosäure (Verbindung 3) mit (ii) einer erfindungsgemäßen Substratverbindung mit direkter Bindung des Fluorophors an den N-Termiunus des Peptids (Verbindung 3; (2)), mit der Zeit nach Umsetzung mit Meerschweinchenleber-Transglutaminase unter den in Beispiel 5 angegebenen Reaktionsbedingungen

### SYNTHESEBEISPIEL 1

### a) Herstellung von N-(2,4-Dinitrophenyl)-1,5-diaminopentan-Hydrochlorid

1,09 g N-(tert.-Butyloxycarbonyl)-1,5-diaminopentan (5,37 mmol) wird in 20 ml Ethanol vorgelegt und bei Raumtemperatur unter Argonatmosphäre nacheinander mit 4,92 ml N-Ethyldiisopropylamin (29 mmol) sowie einer Lösung von 1 g 2,4-Dinitrofluorbenzol (5,37 mmol) in 20 ml Benzol versetzt. Die orange Lösung wird bei Raumtemperatur über Nacht gerührt. Die Lösungsmittel werden im Vakuum entfernt. Der erhaltene Rückstand wird in 150 ml Ethylacetat aufgenommen, jeweils dreimal mit 50 ml 10%iger wässeriger Citronensäurelösung und Wasser gewaschen. Nach Trocknen der organischen Phase mit Na₂SO₄ wird das Lösungsmittel im Vakuum entfernt. Nach erneuter Aufnahme in 20 ml absolutem Dichlormethan und Zusatz von 5 ml einer 4 M Lösung HCl in Dioxan wird für eine Stunde bei Raumtemperatur gerührt. Nach Entfernung des Lösungsmittels im Vakuum erhält man das Produkt als gelbes Pulver.
Ausbeute: 1,94 g (94%)
ESI-MS: 269,0 [M+H]⁺

### b) Herstellung von N^{α}-9-Fluorenylmethoxycarbonyl-N^{δ}-[N-(2,4-dinitrophenyl)]-5-aminopentyl]-L-glutamin (1)

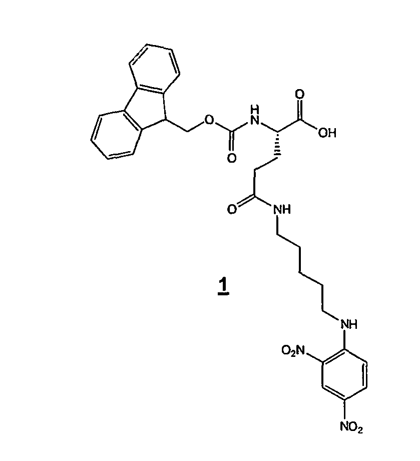

500 mg N^{α}-Fluorenylmethoxycarbonyl-L-glutaminsäure-1-tert.-butylester (1,175 mmol) werden in 10 ml DMF gelöst und mit 382 µl N-Ethyldiisopropylamin (2,24 mmol) versetzt. Unter Argonatmosphäre wird auf -20 °C gekühlt und 161 µl lsobutylchlorformiat (1,175 mmol) zugesetzt. Bei -20°C wird für 30 Minuten gerührt und anschließend eine Lösung aus 326 mg N-[N'-(2,4-Dinitrophenyl)]-1,5-diaminopentan-Hydrochlorid (1,07 mmol) in 5 ml DMF zugegeben. Unter Lichtausschluss wird über Nacht gerührt und währenddessen allmählich auf Raumtemperatur aufgetaut. Das Lösungsmittel wird im Vakuum entfernt und der gelbe, ölige Rückstand in 100 ml Ethylacetat aufgenommen. Es wird jeweils dreimal mit 20 ml 10 %iger wässeriger Citronensäurelösung und Wasser gewaschen. Nach Trocknen der organischen Phase mit Na₂SO₄ wird das Lösungsmittel im Vakuum entfernt. Das erhaltene gelbe Harz, wird in 10 ml absolutem Dichlormethan gelöst und mit 10 ml Trifluoressigsäure versetzt. Bei Raumtemperatur wird für eine Stunde gerührt und danach zur Trockne eingeengt. Die Reingung erfolgt durch Chromatographie an Kieselgel (Säule: 3,9 x 28 cm, Laufmittel:
Dichlormethan/Methanol = 90/10, (v/v) R_{f} = 0,1).
Ausbeute: 630 mg (95 % d. Th.)
ESI-MS: 620,4 [M+H]⁺, 622,4 [M+Na]⁺

### c) Herstellung von 2-Aminobenzoyl-L-asparaainyl-N^{δ}-[N-(2,4-dinitrophenyl)1-5-aminopentyl]-L-glutaminyl-L-glutamyl-L-glutaminyl-L-valinyl-L-serinyl-L-prolinyl-L-leucinyl-L-threoninyl-L-leucinyl-L-leucin-L-lysin (2)

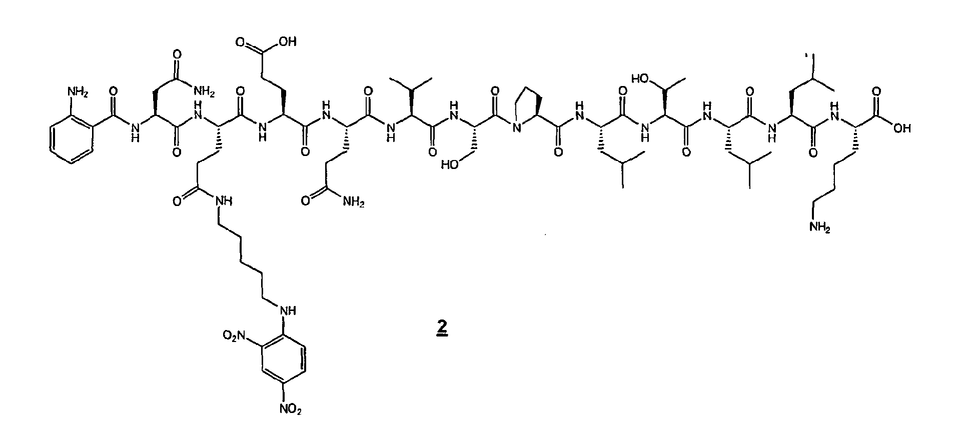

Die Titelverbindung wird nach Standardmethoden der Fmoc-Festphasenpeptidsynthese an einem Polystyrolträger mit der Chlortritylankergruppe dargestellt. Als elfte Aminosäure wird N^{α}-9-Fluorenylmethoxycarbonyl-N^{δ}-[N-(2,4-dinitrophenyl)]-5-aminopentyl]-L-glutamin (1) in die Peptidsequenz eingeführt. Diese modifizierte Aminosäure wird in üblicher Weise durch Aktivierung mit TBTU an das Decapeptid (EQVSPLTLLK) gebunden. Durch abschließende Umsetzung mit N^{α}-9-Fluorenylmethoxycarbonyl-N-β-trityl-L-asparagin wird die Peptidsequenz vervollständigt. Nach Entfernen der N-terminalen Schutzgruppe von dem Dodecapeptid wird 2-(tert.Butyloxycarbonylamino)benzoesäure ebenfalls durch Aktivierung mit TBTU an den N-Terminus des Peptides gebunden. Durch Behandlung des polymeren Trägers mit einer Mischung aus 95% Trifluoressigsäure / 2,5% Trisopropysilan / 2,5% Wasser wird die Titelverbindung als gelber Feststoff erhalten. Die Reinigung erfolgt durch präparative HPLC unter Umkehrphasenbedingungen (Säule: Synergie Max, 4µm, 80 Angström (Phenomenex), 250x21,2 mm, Laufmittel: A-Eluent = 0,1% Trifluoressigsäure in Wasser, B-Eluent = 0,1% Trifluoressigsäure in 9,9% Wasser und 90 % Acetonitril, Gradient: 5% B-Eluent für 15 Minuten. Dann in 95 Minuten auf 100% B-Eluent, Fluss: 8 ml/min. Rₜ = 65,2 min - 68,6 min).
ESI-MS: 1739,9 [M+H]⁺, 1761,9 [M+Na]⁺

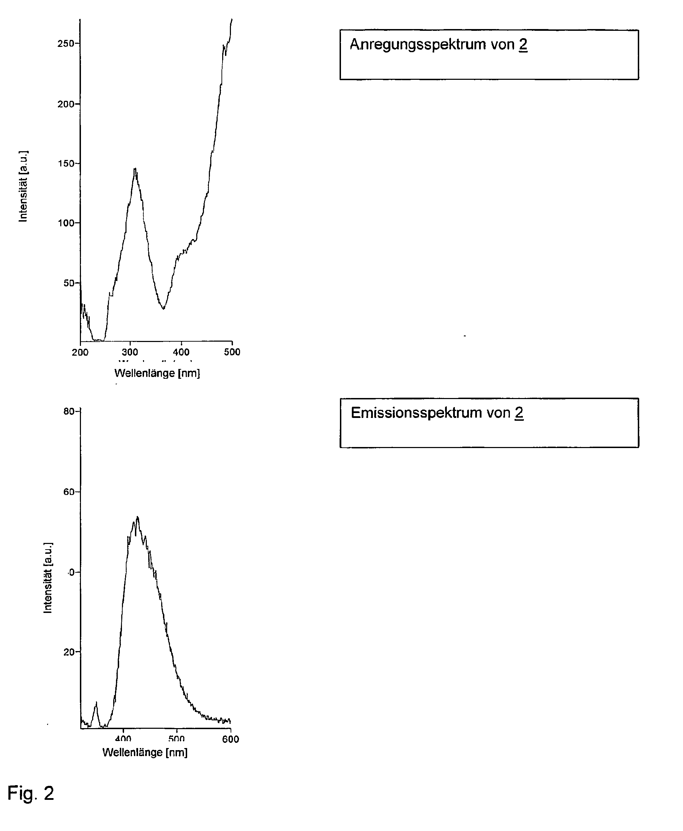

Die Anregungs- und Emissionsspektren von 2 sind in Fig. 2 dargestellt.

### SYNTHESEBEISPIEL 2

### Herstellung von 2-Aminobenzoyl-L-alaninyl-L-prolinyl-N^{δ}-[N-(2,4-dinitrophenyl)]-5-amino-pentyl]-L-glutaminyl-L-glutaminyl-L-glutamyl-alanin (3)

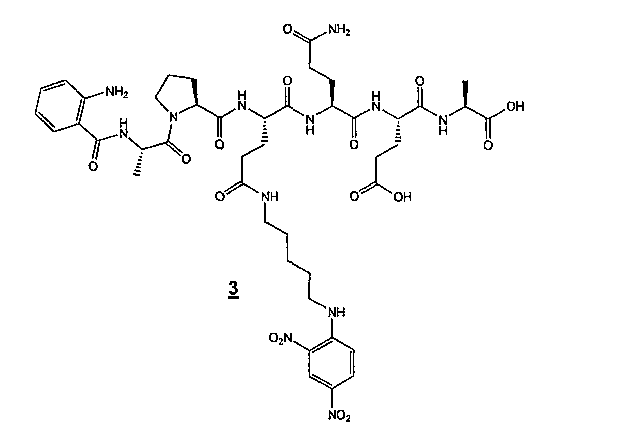

Die Darstellung erfolgt analog der unter Synthesebeispiel 1 angegebenen Weise unter Verwendung der für die obige Peptidsequenz erforderlichen Aminosäuren.
Die Reinigung erfolgt ebenfalls unter den gleichen Bedingungen.
Rₜ= 63-65 min
ESI-MS: 1014,3 [M+H]⁺, 1036,3 [M+Na]⁺

### BEISPIEL 1

### Kinetische Messung von FXIII in Plasmen

### Substratpeptid: Abz-Asn-Glu(CAD-DNP)-Glu-Gln-Val-Ser-Pro-Leu-Thr-Leu-Leu-Lys (2)

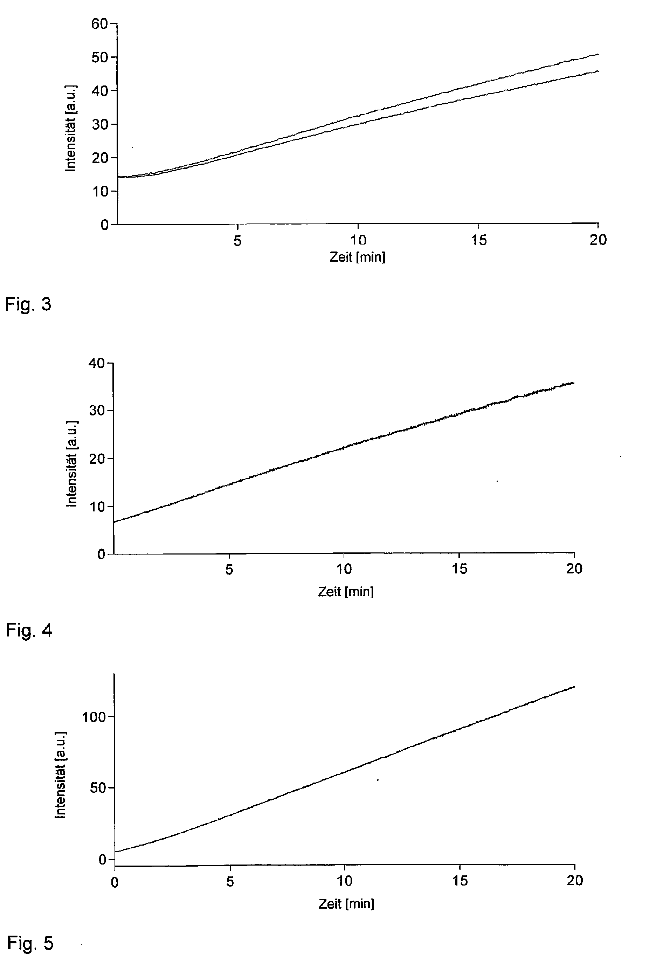

Die Substratsequenz Asn-Gln-Glu-Gln-Val-Ser-Pro-Leu-Thr-Leu-Leu-Lys ist einem natürlichen Substrat des FXIII entnommen und wurde bereits zur kinetischen Messungen von FXIII in der Literatur beschrieben (Kärpäti L. et al., Clin Chem., 46:12, 2000, 1946-55). Durch die erfindungsgemäße Modifikation dieses Peptides kann eine kinetische Messung in Plasmen durchgeführt werden (unterer Graph in Fig. 3). Die Messung wurde in diesem speziellen Fall wie folgt durchgeführt.

| | |
|---|---|
| Ansatzgröße: | 1 ml |
| Substratlösung: | 800 µl |
| Probenvolumen: | 100 µl Citratplasma |
| Thrombinlösung: | 100 µl |
| Ansetzen der Stammlösung: | 7 mg Substrat und 90 mg GPRP-NH₂ in 1 ml DMSO lösen und mit 7 ml Puffer (50 mM Tris-HCl (pH = 7,5), |

| | |
|---|---|
| | 10 mM CaCl₂, 100 mM NaCl, 0,1 % PEG₈₀₀₀) verdünnen. |
| Ansetzen der Substratlösung: | Stammlösung 1/10 (v/v) mit Puffer verdünnen. |
| Durchführung der Messung: | 800 µl Substratlösung mit 100 µl Plasma versetzen, Aktivierung durch Zusatz von 20 NIH Units Thrombin in 100 µl Puffer. Messung bei 37°C. Start der Messung nach 5 min. Exc.: 310 nm Em.: 427 nm, Messung für 10 min |

Führt man die Reaktion in Gegenwart von 5 mM Glycinethylester unter ansonsten identischen Bedingungen durch, wird das Signal zusätzlich verstärkt (oberer Graph in Fig. 3)

### BEISPIEL 2

### Kinetische Messung von gereinigter Transglutaminase 2 (Gewebetransglutaminase)

### Substratpeptid: Abz-Ala-Pro-Glu(CAD-DNP)-Gln-Glu-Ala (3)

Die Substratsequenz Ala-Pro-Gln-Gln-Glu-Ala ist einem natürlichen Substrat der Transglutaminase 2 entnommen (Hohenadl, C., et al., J. Biol. Chem. 1995,:23415-20). Durch die erfindungsgemäße Modifikation dieses Peptides kann eine kinetische Messung von Transglutaminase 2-Aktivität durchgeführt werden. Die Messung wurde in diesem Fall wie folgt durchgeführt.

| | |
|---|---|
| Ansatzgröße: | 1 ml |
| Abz-Ala-Pro-Glu(CAD-DNP)-Gln-Glu-Ala (3): | 50 µM (vorgelöst in DMSO) |
| rhHis₆TG2: | 0,25 µM |
| Puffer: | 50 mM Tris-HCl, pH 7,5 , 10 mM CaCl₂, 100 mM NaCl, 0,1% PEG |
| DMSO-Gehalt im Ansatz: | 6 % |
| Messbedingungen: | |
| | Exc.: 310 nm |
| | Em.: 427 nm, Messung für 20 min |

Das Ergebnis der Messung ist in Fig. 4 graphisch dargestellt.

### BEISPIEL 3

### Kinetische Messung von Meerschweinchenleberhomogenisat

Meerschweinchenleber enthält eine Transglutaminase, die in der wissenschaftlichen Literatur aufgrund ihrer starken Homologie zu humaner Transglutaminase 2 häufig als Modellenzym eingesetzt wird. Aufgrund dieser Homologie kann die Aktivität dieses Enzyms ebenfalls mit Verbindung 3 gemessen werden.

Das Homogenisat wird hergestellt nach Leblanc et al. (Leblanc et al., Protein Expr. Purif., 1999, 89-95).

Von diesem Homogenisat werden 20 µl zu 980 µl einer Lösung gegeben, so dass folgende Zusammensetzung im Ansatz vorliegt:

| | |
|---|---|
| Ansatzgröße: | 1 ml |
| Abz-Ala-Pro-Glu(CAD-DNP)-Gln-Glu-Ala (3): | 50 µM (vorgelöst in DMSO) |
| Meerschweinchenleberhomogenisat: | 20 µl |
| Puffer: | 50 mM Tris-HCl, pH 7,5 , 10 mM CaCl₂, 100 mM NaCl, 0,1% PEG |
| DMSO-Gehalt im Ansatz: | 6 % |
| Messbedingungen: | |
| | Exc.: 310 nm |
| | Em.: 427 nm, Messung für 20 min |

Das Ergebnis der Messung ist in Fig. 5 graphisch dargestellt.

### BEISPIEL 4

### Selektivität der Messungen verschiedener Transglutaminasen

### 1. Ansatz: Substratpeptid: Abz-Ala-Pro-Glu(CAD-DNP)-Gln-Glu-Ala (3), Transglutaminase: Faktor XIII

| | |
|---|---|
| Ansatzgröße: | 1 ml |
| Abz-Ala-Pro-Glu(CAD-DNP)-Gln-Glu-Ala (3): | 50 µM (vorgelöst in DMSO) |
| Faktor XIII: | 100 µl Citratplasma |
| Puffer: | 50 mM Tris-HCl, pH 7,5 , 10 mM CaCl₂, 100 mM NaCl, 0,1% PEG |
| DMSO-Gehalt im Ansatz: | 6 % |
| Messbedingungen: | |
| | Exc.: 310 nm |
| | Em.: 427 nm, Messung für 20 min |
| Ergebnis: kein Anstieg der Fluoreszenz | |

### 2. Ansatz: Abz-Asn-Glu(CAD-DNP)-Glu-Gln-Val-Ser-Pro-Leu-Thr-Leu-Leu-Lys (2), Transglutaminase: gereinigte rekombinante humane Transglutaminase 2

| | |
|---|---|
| Ansatzgröße: | 1 ml |
| Abz-Asn-Glu(CAD-DNP)-Glu-Gln-Val-Ser-Pro-Leu-Thr-Leu-Leu-Lys (2): | 50 µM |
| rhHis₆TG2: | 0,25 µM |
| Puffer: | 50 mM Tris-HCl, pH 7,5 , 10 mM CaCl₂, 100 mM NaCl, 0,1% PEG |
| DMSO Gehalt im Ansatz: | 6 % |
| Messbedingungen: | |
| | Exc.: 310 nm |
| | Em.: 427 nm, Messung für 20 min |
| Ergebnis: kein Anstieg der Fluoreszenz | |

### BEISPIEL 5

### Vergleich von Transglutaminasepeptidsubstraten

Für den Vergleich verschiedener mit fluoreszierenden Gruppen markierter Transglutaminasepeptidsubstrate wurden kinetische Messungen unter identischen Bedingungen durchgeführt. Dazu wurden zu 900 µl einer thrombinhaltigen 50 µM Lösung des Peptids 100 µl Humanplasma zugesetzt. Diese Bedingungen entsprechen denen, die bei heutzutage üblichen Faktor XIII - Messungen (wie bei dem Marktführer: Berichrom) zum Einsatz kommen. Die Verbindung 1 (DNS-Ahx-Gln(CAD-DNP)-Glu-lle-Val-OH, siehe Parameswaran et al.) lieferte unter diesen klinisch relevanten Bedingungen keine messbare Steigerung der Fluoreszenz als Messgröße. Auch die Verwendung von Abz als Fluorophor (Abz-Ahx-Glu(CAD-DNP)-Gln-Ile-Val, (Parameswaran et al.,

Seite 10316, Fig. 7) lieferte unter den identischen Bedingungen keine messbare Steigerung der Fluoreszenz (die Daten wurden nicht abgebildet, siehe Parameswaran et al.).

Um die Substrateigenschaften gegenüber den Substraten des Standes der Technik zu verbessern und somit klinisch relevante Messungen zu ermöglichen, wurde ausgehend von Verbindung 1 durch routinemäßiges Experimentieren das Peptidrückgrat so verändert, dass ein Substrat entstand, das durch FXIII schneller umgesetzt werden sollte (DNS-Ahx-Glu(CAD-DNP)-Glu-Gln-Val-Ser-Pro-Leu-Thr-Leu-Leu-Lys, 2). Das molekulare Design von 2 zielte darauf ab, unter Erhalt der in Parameswaran et al. offengelegten Teilstruktur (DNS-Ahx-Glu(CAD-DNP) durch den bloßen Austausch der Aminosäuren in Richtung C-Terminus, einem dem Fachmann bekannten, guten Substrat des Faktor XIII (Asn-Gln-Glu-Gln-Val-Ser-Pro-Leu-Thr-Leu-Leu-Lys, Kärpäti et al., Clin. Chem., 2000, 1946 - 55, Substrat-Glutamin unterstrichen) zu entsprechen. Die genannte Teilstruktur enthält beide für die erforderliche Fluoreszenzsteigung relevanten Gruppen (Fluorophor und Quencher). Wie in Fig. 6 zu erkennen ist, führte diese Modifikation jedoch nicht zu einer Verbesserung.

Gegenüber dem Peptid, das in der Literatur (Kärpäti et al.) beschrieben wird, ist die Verbindung 2 um eine Aminosäure am N-Terminus verkürzt. Dahinter stand die Absicht, die im Stand der Technik bekannte und oben beschriebene fluorogene Teilstruktur zu erhalten. Es ist aber bekannt, dass gerade die Aminsäuren, die der Bindungsstelle von Enzymen direkt benachbart sind, besonders großen Einfluss auf die Substrateigenschaften von Peptiden, bzw. Proteinen haben. Deshalb wurde in einer weiteren Modifikation von Verbindung 1 die Verbindung 3 hergestellt. (DNS-Ahx-Asn-Glu(CAD-DNP)-Glu-Gln-Val-Ser-Pro-Leu-Thr-Leu-Leu-Lys, 3). Wie Fig. 6 zeigt, führt auch diese Modifikation nicht zu dem gewünschten Ergebnis.

Überraschenderweise wurde anhand verschiedener Peptide und Transglutaminasen gezeigt, dass durch die Entfernung des Aminohexansäurespacers zwischen der N-terminalen Aminosäure und dem Fluorophor eine wesentliche Verbesserung der Fluoreszenzsteigerung erreicht werden kann. Beispielhaft wird dies im Vergleich in Fig. 6 anhand der Reaktion von 100 µl humanem Plasma (durch Thrombin aktiviert) und einer 50 µM Lösung des Peptides Abz-Asn-Glu(CAD-DNP)-Glu-Gln-Val-Ser-Pro-Leu-Thr-Leu-Leu-Lys (Verbindung 2)) gezeigt. Dem Vergleich der Strukturen und der Fluoreszenzdaten ist zu entnehmen, dass diese Wirkung auch für andere Transglutaminasen und andere Peptide Gültigkeit hat.

Das erfindungsgemäße Verfahren bietet breite Anwendungsmöglichkeiten sowohl in der medizinischen Forschung als auch in der klinischen Entwicklung.

Transglutaminasen sind im menschlichen Körper an zahlreichen Prozessen beteiligt und können dementsprechend in einer Vielzahl von Krankheiten eine Rolle spielen. Aus der wissenschaftlichen Literatur gilt eine Beteiligung der Gewebe-Transglutaminase an der Zöliakie als gesichert. Die Datenlage bezüglich eines Einflusses auf Krankheiten wie Katarakt, Arteriosklerose und insbesondere neurodegenerative Erkrankungen (Chorea Huntington und Alzheimersche Krankheit) verdichten sich.

Auch der Blutgerinnungsfaktor XIII steht im Zusammenhang mit vielen ernsthaften Erkrankungen (siehe Klinische Aspekte des Faktor XIII Mangels, Karger Verlag, 1999, Hrsg., R. Ebbing, R. Seitz, G. Wozinak). Der genetisch bedingte Mangel an Transglutaminase 1 führt zur so genannten lamellären Ichthyose. Hier dient der Aktivitätsnachweis als diagnostisches Kriterium für diese Krankheit (Raghunath et al., Arch Dermatol Res,1998; 290:21-627).

Anwendungsmöglichkeiten des vorliegenden Verfahrens liegen deshalb sowohl in der medizinischen Grundlagenforschung dieser und anderer Transglutaminase-abhängigen Krankheiten als auch in der klinischen Diagnostik.

Eine weitere wesentliche Anwendungsmöglichkeit ist die klinische Entwicklung neuer Medikamente. Das vorliegende Verfahren ist geeignet über einen weiten Aktivitätsbereich sehr genau und zuverlässig zu messen. Dadurch und durch den einfachen und stabilen Aufbau des Assay-Systems, eignet sich das Verfahren in besonderer Weise zum Einsatz in sogenannten Hochdurchsatzscreenings (HTS, *high throughput screening*)*,* bei denen die Wirkung einer großen Anzahl von Wirkstoffkandidaten auf die Enzyme (hier Transglutaminasen) parallel getestet werden.

## Patentansprüche

1. Verfahren zur fluoreszenzbasierenden kinetischen Messung von Transglutaminase-Aktivität, **dadurch gekennzeichnet, dass** die Transglutaminase mit einem modifizierten Peptidsubstrat, bei dem ein Fluorophor direkt an den N-Terminus des Peptids gebunden ist und ein Glutaminsäurerest in 5-Position einen Fluoreszenzquencher trägt, welcher über einen Spacer, der eine Aminfunktion enthält, unter Ausbildung einer Amidbindung an die 5-Position gebunden ist, umgesetzt wird, so dass durch die Aktivität der Transglutaminase der Fluoreszenzquencher abgespalten wird, und die resultierende Fluoreszenz des Restmoleküls gemessen wird.

2. Verfahren nach Anspruch 1, **dadurch gekennzeichnet, dass** das Fluorophor ein am Benzolring substituiertes Benzoesäurederivat ist.

3. Verfahren nach Anspruch 1 oder 2, **dadurch gekennzeichnet, dass** das Fluorophor 2-Aminobenzoesäure oder N-Methyl-2-aminobenzoesäure ist.

4. Verfahren nach Anspruch 3, **dadurch gekennzeichnet, dass** das Peptidsubstrat die folgende Formel I aufweiset, 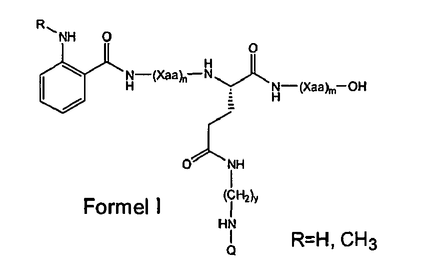
worin Xaa ein beliebige Aminosäure oder ein Aminosäurederivat davon ist;
n und und m unabhängig voneinander 0 bis 10 sind,
y 1 bis 6, vorzugsweise 5, ist,
Q ein Fluoreszenzquencher ist,
R H oder CH₃ ist.

5. Verfahren nach Anspruch 4, **dadurch gekennzeichnet, dass** das Peptidsubstrat aus der Gruppe, bestehend aus den Verbindungen Abz-Asn-Glu((CH₂)_{y}-NH-Q)-Glu-Gln-Val-Ser-Pro-Leu-Thr-Leu-Leu-Lys, Abz-Pyr-Ala-Glu((CH₂)_{y}-NH-Q)-Gln-Ile-Val, Abz-Ala-Pro-Glu((CH₂)_{y}-NH-Q)-Gln-Glu-Ala und N-Methyl-Abz-Derivaten davon, ausgewählt ist.

6. Verfahren nach einem der Ansprüche 1-5, **dadurch gekennzeichnet, dass** der Fluoreszenzquencher eine 2,4-Dinitrophenyl- oder 3-Nitrotyrosingruppe enthält.

7. Verfahren nach einem der Ansprüche 1-6, **dadurch gekennzeichnet, dass** die Transglutaminase eine Säuger-Transglutaminase, insbesondere eine humane Transglutaminase, ist.

8. Verfahren nach Anspruch 7, **dadurch gekennzeichnet, dass** die Transglutaminase humane Transglutaminase 2 oder FXIII ist.

9. Verfahren nach einem der Ansprüche 1-8, **dadurch gekennzeichnet, dass** die Transglutaminase ein Blutgerinnungsfaktor ist und Gly-Pro-Arg-Pro-amid als Fibrinaggregationshemmer verwendet wird.

10. Verfahren nach Anspruch 8 oder 9, **dadurch gekennzeichnet, dass** die Transglutaminase Blutgerinnungsfaktor FXIII ist und das FXIII durch Thrombin aktiviert wird.

11. Verfahren nach einem der Ansprüche 1-10, **dadurch gekennzeichnet, dass** ein Amin zugesetzt wird.

12. Verfahren nach Anspruch 11, **dadurch gekennzeichnet, dass** das Amin ein Glycinmethyl- oder -ethylester, Ethanolamin, Diaminoethan oder ein Derivat von 1,5-Diaminopentan ist.

13. Verfahren nach einem der Ansprüche 1-12, **dadurch gekennzeichnet, dass** die Transglutaminase-Aktivität sowohl in gereinigter Form als auch in ungereinigter Form, z. B. in Zellhomogenisaten, gemessen werden kann.

14. Verfahren nach einem der Ansprüche 1-13, **dadurch gekennzeichnet, dass** die Messung der Aktivität einer Transglutaminase unabhängig von der Gegenwart anderer Transglutaminasen erfolgen kann.

15. Verfahren nach einem der Ansprüche 1-14, **dadurch gekennzeichnet, dass** die Aktivitätsmessung sowohl als Einzelmessung als auch als parallele Messung in einer für ein parallelisiertes Messverfahren geeigneten Vorrichtung durchgeführt werden kann.

16. Verfahren nach Anspruch 15, **dadurch gekennzeichnet, dass** die Messung in einer Mikrotiterplatte durchgeführt wird.

17. Verfahren zur Identifizierung von Verbindungen, die eine Transglutaminase-Aktivität modulieren, umfassend das Inkontaktbringen von wenigstens eine Verbindung mit einer Transglutaminase und die anschließende Messung der Transglutaminase-Aktivität gemäß einem der Verfahren der Anspruche 1 bis 16.

18. Verfahren nach Anspruch 17, bei dem Verbindungen identifiziert werden, die eine Transglutaminase-Aktivität verstärken oder verringern, vorzugsweise die Transglutaminase-Aktivität verringern.

19. Fluoreszenzmarkiertes Peptidsubstrat für Transglutaminasen, **dadurch gekennzeichnet, dass** ein Fluorophor direkt an den N-Terminus des Peptids gebunden ist und ein Glutaminsäurerest in 5-Position einen Fluoreszenzquencher trägt, welcher über einen Spacer, der eine Aminfunktion enthält, unter Ausbildung einer Amidbindung an die 5-Position gebunden ist.

20. Peptidsubstrat nach Anspruch 19, **dadurch gekennzeichnet, dass** das Fluorophor ein am Benzolring substituiertes Benzoesäurederivat ist.

21. Peptidsubstrat nach Anspruch 20, **dadurch gekennzeichnet, dass** das Fluorophor 2-Aminobenzoesäure oder N-Methyl-2-aminobenzoesäure ist.

22. Peptidsubstrat nach Anspruch 21, **dadurch gekennzeichnet, dass** das Peptidsubstrat die folgende Formel I aufweiset, 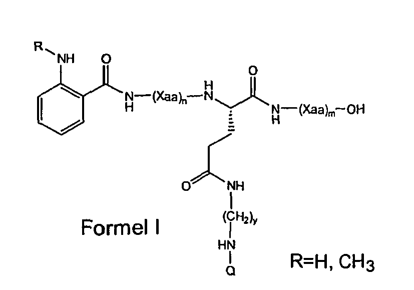
worin Xaa ein beliebige Aminosäure oder ein Aminosäurederivat davon ist,
n und und m unabhängig voneinander 0 bis 10 sind,
y 1 bis 6, vorzugsweise 5, ist,
Q ein Fluoreszenzquencher ist,
R H oder CH₃ ist.

23. Peptidsubstrat nach Anspruch 22, **dadurch gekennzeichnet, dass** das Peptidsubstrat aus der Gruppe, bestehend aus den Verbindungen Abz-Asn-Glu((CH₂)_{y}-NH-Q)-Glu-Gln-Val-Ser-Pro-Leu-Thr-Leu-Leu-Lys, Abz-Pyr-Ala-Glu((CH₂)_{y}NH-Q)-Gln-Ile-Val, Abz-Ala-Pro-Glu((CH₂)_{y}-NH-Q)-Gln-Glu-Ala und N-Methyl-Abz-Derivaten davon, ausgewählt ist.

24. Peptidsubstrat nach einem der Ansprüche 19-23, **dadurch gekennzeichnet, dass** der Fluoreszenzquencher eine 2,4-Dinitrophenyl- oder 3-Nitrotyrosingruppe enthält.

25. Verwendung eines Peptidsubstrats gemäß einem der Ansprüche 19 bis 23 in einem Verfahren zur fluoreszenbasierenden kinetische Messung von Transglutaminase-aktivität.

## Claims

1. Method of fluorescence-based kinetic measurement of transglutaminase activity, **characterized in, that** the transglutaminase is reacted with a modified peptide substrate in which a fluorophore is bound directly to the N-terminus of the peptide and a glutamic acid residue in 5-position has a fluorescence quencher which is bound to the 5-position via a spacer having an amine function resulting in an amide bond, such that the fluorescence quencher is cleaved by the activity of the transglutaminase and the resulting fluorescence of the remaining molecule is measured.

2. Method according to claim 1, **characterized in, that** the fluorophore is a benzoic acid derivative substituted at the benzene ring.

3. Method according to claims 1 or 2, **characterized in, that** the fluorophore is 2-aminobenzoic acid or N-methyl-2-aminobenzoic acid.

4. Method according to claim 3, **characterized in, that** the peptide substrate has the following formula I 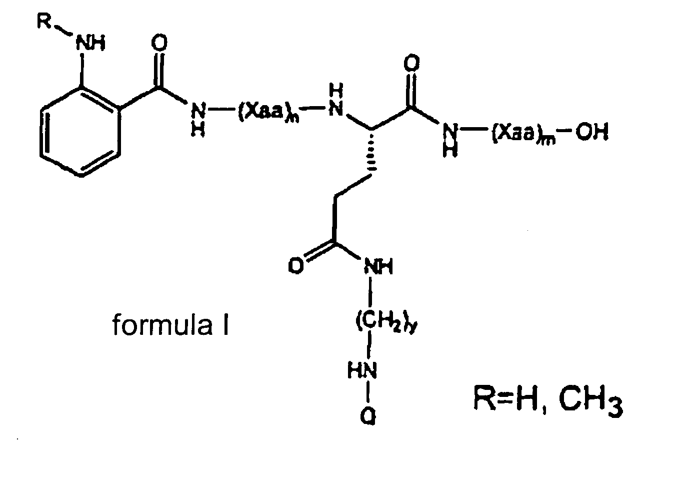
wherein Xaa is any amino acid or an amino acid derivative thereof,
n and m are independently from each other 0 to 10,
y is 1 to 6, preferably 5,
Q is a fluorescence quencher,
R is H or CH₃.

5. Method according to claim 4, **characterized in, that** the peptide substrate is selected from the group consisting of the compounds Abz-Asn-Glu((CH₂)_{y}-NH-Q)-Glu-Gln-Val-Ser-Pro-Leu-Thr-Leu-Leu-Lys, Abz-Pyr-Ala-Glu((CH₂)_{y}-NH-Q)-Gln-Ile-Val, Abz-Ala-Pro-Glu((CH₂)_{y}-NH-Q)-Gln-Glu-Ala and N-methyl-Abz derivatives thereof.

6. Method according to one of the claims 1-5, **characterized in, that** the fluorescence quencher contains a 2,4-dinitrophenyl or 3-nitrotyrosine group.

7. Method according to one of the claims 1-6, **characterized in, that** the transglutaminase is a mammalian transglutaminase, especially a human transglutaminase.

8. Method according to claim 7, **characterized in, that** the transglutaminase is human transglutaminase 2 or FXIII.

9. Method according to one of the claims 1-8, **characterized in, that** the transglutaminase is a blood clotting factor and that Gly-Pro-Arg-Pro-amide is used as fibrin aggregation inhibitor.

10. Method according to claim 8 or 9, **characterized in, that** the transglutaminase is blood clotting factor FXIII and that the FXIII is activated by thrombin.

11. Method according to one of the claims 1-10, **characterized in, that** an amine is added.

12. Method according to claim 11, **characterized in, that** the amine is a methyl- or ethylester of glycine, ethanolamine, diaminoethane or a derivative of 1,5-diaminopentane.

13. Method according to one of the claims 1-12, **characterized in, that** the transglutaminase activity is measurable in both purified form and unpurified form, e.g. in cell homogenisates.

14. Method according to one of the claims 1-13, **characterized in, that** the activity measurement of a transglutaminase is feasible independently of the presence of other transglutaminases.

15. Method according to one of the claims 1-14, **characterized in, that** the activity measurement can be carried out as both individual measurement and as parallel measurement in a device suitable for a parallel measuring method.

16. Method according to claim 15, **characterized in, that** the measurement is carried out in a microtiter plate.

17. Method of identifying compounds which modulate a transglutaminase activity, comprising contacting of at least one compound with a transglutaminase and subsequent measuring of the transglutaminase activity according to a method of the claims 1 to 16.

18. Method according to claim 17, in which compounds are identified that increase or reduce a transglutaminase activity, preferably reduce the transglutaminase activity.

19. Fluorescence labeled peptide substrate for transglutaminases, **characterized in, that** a fluorophore is bound directly to the N-terminus of the peptide and a glutamic acid residue in 5-position has a fluorescence quencher which is bound to the 5-position via a spacer having an amine function resulting in an amide bond.

20. Peptide substrate according to claim 19, **characterized in, that** the fluorophore is a benzoic acid derivative substituted at the benzene ring.

21. Peptide substrate according to claim 20, **characterized in, that** the fluorophore is 2-aminobenzoic acid or N-methyl-2-aminobenzoic acid.

22. Peptide substrate according to claim 21, **characterized in, that** the peptide substrate has the following formula I 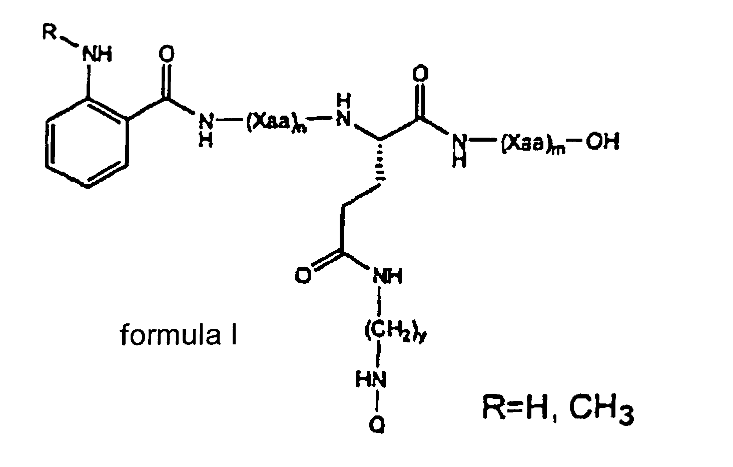
wherein Xaa is any amino acid or an amino acid derivative thereof,
n and m are independently from each other 0 to 10,
y is 1 to 6, preferably 5,
Q is a fluorescence quencher,
R is H or CH₃.

23. Peptide substrate according to claim 22, **characterized in, that** the peptide substrate is selected from the group consisting of the compounds Abz-Asn-Glu((CH₂)_{y}-NH-Q)-Glu-Gln-Val-Ser-Pro-Leu-Thr-Leu-Leu-Lys, Abz-Pyr-Ala-Glu((CH₂)_{y}-NH-Q)-Gln-Ile-Val, Abz-Ala-Pro-Glu((CH₂)_{y}-NH-Q)-Gln-Glu-Ala and N-methyl-Abz derivatives thereof.

24. Peptide substrate according to one of the claims 19-23, **characterized in, that** the fluorescence quencher contains a 2,4-dinitrophenyl or 3-nitrotyrosine group.

25. Use of a peptide substrate according to one of the claims 19 to 23 in a method of fluorescence-based kinetic measurement of transglutaminase activity.

## Revendications

1. Procédé pour la mesure cinétique basée sur le fluorescence de l'activité de la transglutaminase, **caractérisé en ce que** la transglutaminase est transformée avec un substrat peptidique modifié , dans lequel un fluophore est lié directement au terminus N du peptide et un résidu de l'acide glutamique porte dans la position 5 un désactiveur de fluorescence , lequel est lié via un espaceur, comportant une fonction d'amine, à la position 5 en formant une liaison d'amide , de manière à ce que le désactiveur de fluorescence est séparé grâce à l'activité de la transglutaminase et **en ce que** la fluorescence du molécule résiduel qui en résulte, est mesurée.

2. Procédé selon la revendication 1, **caractérisé en ce qu'**un fluorophore est un dérivé de l'acide benzoïque substitué sur l'anneau benzénique.

3. Procédé selon les revendications 1 ou 2, **caractérisé en ce que** le fluorophore est de l'acide 2 -aminobenzoïque ou de l'acide N - méthyle - 2 - aminobenzoïque.

4. Procédé selon la revendication 3, **caractérisé en ce que** le substrat peptidique présente la formule I suivante : 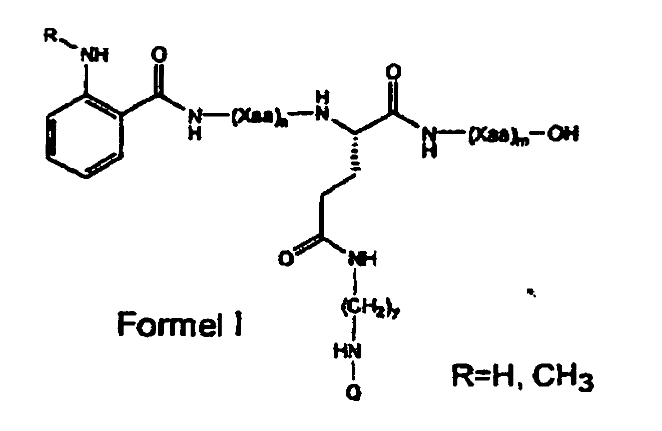
où Xaa est un acide aminé quelconque ou un dérivé d'acide miné de cette formule,
n et m sont 0 à 10 indépendant l'un de l'autre,
y est 1 à 6, de préférence 5,
Q est un désactiveur de fluorescence,
R est H ou CH₃.

5. Procédé selon la revendication 4, **caractérisé en ce que** le substrat peptidique est sélectionné du groupe composé des composés Abz - Asn - Glu ((CH₂)_{y} - NH-Q)-Glu-Gln-Val-Ser-Pro-Leu-Thr - Leu - Leu - Lys, Abz - Pyr - Ala - Glu ((CH₂)_{y} - NH - Q) - Gln - Ile - Val, Abz - Ala - Pro - Glu ((CH₂)_{y} - NH - Q ) - Gln - Glu - Ala et des dérivés N - méthyle - Abz de ces composés ci-dessus.

6. Procédé selon une des revendications 1 à 5, **caractérisé en ce que** le désactiveur de fluorescence comporte un groupe 2,3 - dinitrophényle ou de 3 - nitrotyrosine.

7. Procédé selon une des revendications 1 à 6, **caractérisé en ce que** la transglutaminase est une transglutaminase de mammifère, notamment une transglutaminase humaine.

8. Procédé selon la revendication 7, **caractérisé en ce que** la transglutaminase est une transglutaminase 2 ou FXIII.

9. Procédé selon une des revendications 1 à 8, **caractérisé en ce que** la transglutaminase est un facteur de coagulation sanguine et que l'amide de Gly - Pro - Arg - Pro est utilisé comme frein de l'agrégation de fibrine.

10. Procédé selon une des revendications 8 ou 9, **caractérisé en ce que** la transglutaminase est le facteur de coagulation sanguine FXIII et que le FXIII est activé par la trombine.

11. Procédé selon une des revendications 1 à 10, **caractérisé en ce que** l'on ajoute une amine.

12. Procédé selon la revendication 11, **caractérisé en ce que** l'amine est un ester méthylique de la glycine ou un ester éthylique , une éthanolamine , une diaminoéthane ou un dérivé de diaminopentane .

13. Procédé selon une des revendications 1 à 12, **caractérisé en ce que** l'activité de la transglutaminase peut être mesurée à l'état purifié ou à l'état non purifié par exemple dans des broyats cellulaires.

14. Procédé selon une des revendications 1 à 13, **caractérisé en ce que** la mesure de l'activité d'une transglutaminase est effectuée indépendamment de la présence d'autres transglutaminases.

15. Procédé selon une des revendications 1 à 14, **caractérisé en ce que** la mesure de l'activité est effectuée en tant que mesure individuelle et en tant que mesure parallèle dans un dispositif adapté à un procédé de mesure parallélisée.

16. Procédé selon la revendication 15, **caractérisé en ce que** la mesure est effectuée dans une plaque de microtitrage

17. Procédé pour l'identification de composés, qui modulent une activité de transglutaminase , comportant la mise en contact d'au moins un composé avec une transglutaminase et la mesure ultérieure de l'activité de transglutaminase selon un des procédés des revendications 1 à 16.

18. Procédé selon la revendication 17, dans lequel on identifie des composés qui amplifient ou diminuent une activité de transglutaminase , de préférence diminuent l'activité de glutaminase.

19. Substrat peptidique pour les transglutaminases comportant des marques fluorescentes , **caractérisé en ce qu'**un fluorophore est lié directement au terminus N du peptide et porte un résidu de l'acide glutamique dans la position 5 d'un séactiveur de fluorescence , lequel désactiveur est lié via un espacer, comportant une fonction d'amine, à la position 5 en formant une liaison d'amide.

20. Substrat peptidique selon la revendication 19, **caractérisé en ce que** le fluorophore est un dérivé de l'acide benzoïque substitué sur l'anneau benzénique.

21. Substrat peptidique selon la revendication 20, **caractérisé en ce que** le fluorophore est de l'acide 2 -aminobenzoïque ou de l'acide N - méthyle - 2 - aminobenzoïque.

22. Substrat peptidique selon la revendication 21, **caractérisé en ce que** le substrat peptidique présente la formule suivants : 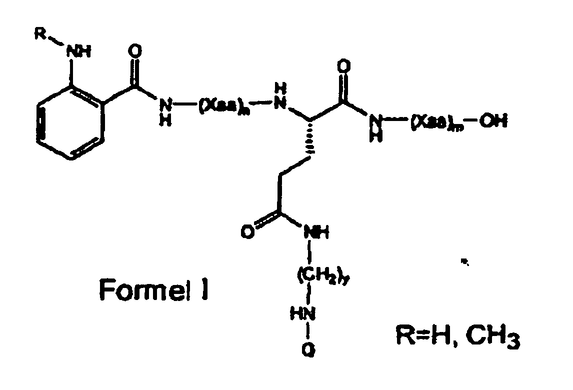
où Xaa est un acide aminé quelconque ou un dérivé d'acide miné de cette formule,
n et m sont 0 à 10 indépendant l'un de l'autre,
y est 1 à 6, de préférence 5,
Q est un désactiveur de fluorescence,
R est H ou CH₃.

23. Substrat peptidique selon la revendication 22, **caractérisé en ce que** le substrat peptidique est sélectionné du groupe composé des composés Abz - Asn - Glu ((CH₂)_{y} - NH - Q) - Glu - Gln - Val - Ser - Pro - Leu - Thr - Leu - Leu - Lys, Abz - Pyr - Ala - Glu ((CH₂)_{y} - NH - Q) - Gln - Ile - Val , Abz - Ala - Pro - Glu ((CH₂)_{y} - NH - Q ) - Gln - Glu - Ala et des dérivés N - méthyle - Abz de ces composés ci-dessus.

24. Substrat peptidique selon une des revendications 19 à 23, **caractérisé en ce que** le désactiveur de fluorescence comporte un groupe 2,3 - dinitrophényle ou de 3 - nitrotyrosine.

25. Utilisation d'un substrat peptidique selon une des revendications 19 à 23 dans un procédé pour la mesure cinétique basée sur le fluorescence de l'activité de la transglutaminase.
